# EUROPEAN PATENT SPECIFICATION

(11) **EP 2 035 040 B1**
(45) Date of publication and mention of the grant of the patent: **01.05.2013**
(21) Application number: 07764310.4
(22) Date of filing: 20.06.2007
(51) Int. Cl.: A61K 9/19, A61K 47/40, A61K 47/48, A61K 31/282, A61K 33/24

(54) **PHARMACEUTICAL COMPOSITION FOR ADMINISTRATION BY INJECTION**
PHARMAZEUTISCHE ZUSAMMENSETZUNG ZUR VERABREICHUNG DURCH INJEKTION
PRÉPARATION PHARMACEUTIQUE INJECTABLE

(30) Priority: 20.06.2006 CZ 20060403
(43) Date of publication of application: 18.03.2009
(73) Proprietor: Pliva-Lachema A.S., 621 33 Brno (CZ)
(72) Inventor: FRANC, Ales, 638 00 Brno (CZ); SOVA, Petr, 500 02 Hradec Králové (CZ)
(74) Representative: Pavlica, Tomas
(86) International application number: PCT/CZ2007/000059
(87) International publication number: WO 2007/147372

(56) References cited:
- WO-A-99/61450
- WO-A-99/61451
- WO-A-2006/029579
- WO-A-2006/095016
- US-A- 4 696 918
- US-A- 5 238 955
- US-A- 6 136 336
- KIZU RYOICHI ET AL: "Significance of water solubility in the gastrointestinal absorption of trans-bis(n-valerato)(1R,2R-cyclohexanedia mine)(oxalato)platinum(IV), an orally active antitumor platinum complex, and its analogs" ANTI-CANCER DRUGS, RAPID COMMUNICATIONS, OXFORD, GB, vol. 9 (2), 1998, pages 167-174, XP008087321 ISSN: 0959-4973
- PROCHAZKA ET AL: "Apoptosis and inhibition of gap-junctional intercellular communication induced by LA-12, a novel hydrophobic platinum(IV) complex" ARCHIVES OF BIOCHEMISTRY AND BIOPHYSICS, NEW YORK, US, US, vol. 462, no. 1, 18 May 2007 (2007-05-18), pages 54-61, XP022083633 ISSN: 0003-9861

## Description

### Field of the Invention

The invention relates to a pharmaceutical composition for administration by injections, containing as active substance a complex of tetravalent platinum of limited solubility in water and enabling systemic as well as local application of the said complex to the organism.

### Background of the Invention

Platinum complexes are generally known as effective substances having a broad spectrum of antitumor effect and they are thus utilized in the treatment of many of tumor diseases. So far, the therapeutic practice has used only complexes of bivalent platinum, especially cisplatinum, carboplatinum or oxaliplatinum. It has been found that some complexes of tetravalent platinum exhibit the same, or even higher, antitumor effect in comparison with that of bivalent platinum complexes and, moreover, they are less toxic. Specific complexes of tetravalent platinum were disclosed as novel chemical compounds in EP 0 328 274, EP 0 423 707 and WO 99/61451 (PCT/CZ99/00015).

However, complexes of tetravalent platinum in general are very poorly soluble in water, which represents a serious problem in cases where it is necessary to prepare their liquid, especially injection, drug form. Whereas the solubility of cisplatinum, carboplatinum and oxaliplatinum in water is 0.3 mg/ml, 17 mg/ml and 8 mg/ml, respectively, the solubility of the tetravalent platinum complex LA-12 in water is only 0.03 mg/ml. The solubility of tetravalent platinum complexes in water can be increased according to WO 99/61451 (PCT/CZ99/00015) which patent document describes the preparation of drug forms of specific tetravalent platinum complexes in the form of their inclusion complexes with cyclodextrins. According to the mentioned document, these inclusion complexes are obtained by reaction of cyclodextrins with complexes of tetravalent platinum in an organic solvent followed by freeze-drying, and are used for oral administration. However, disadvantage of this approach is that the amount of cyclodextrin used limits significantly the content of the tetravalent platinum complex present in the oral drug form. Thus, the obtained oral drug form is relatively voluminous and is difficult to swallow which precludes oral administration of greater amounts of tetravalent platinum complexes in a single dose. This situation could be solved by application of a relatively well water-soluble complex of cyclodextrin with tetravalent platinum complex in the form of injection. This, however, considerably complicates the relatively complicated and expensive preparation of the inclusion complex of cyclodextrin with the tetravalent platinum complex which so far requires the presence of an organic solvent.

Therefore, the aim of the invention is to find a simpler method of preparation of inclusion complexes of cyclodextrin with tetravalent platinum complexes and to provide a pharmaceutical composition comprising the thus-prepared inclusion complex and being applicable in the injection form.

Surprisingly, it has been found that inclusion complexes of specific tetravalent platinum complexes with cyclodextrins can be simply prepared solely in an aqueous medium, which represents a significant simplification not only of their preparation but also of the preparation of injectable pharmaceutical composition.

### Summary of the invention

The invention relates to a method for manufacturing a pharmaceutical composition for administration by injection consisting of an aqueous solution of an inclusion complex of a platinum complex of formula II 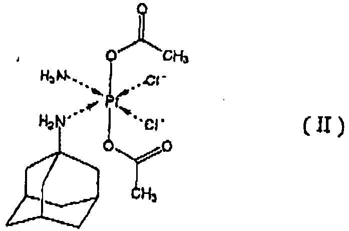 with at least one cyclodextrin and/or at least one of alpha-, beta- and gamma-cyclodextrin alkylated derivatives and optionally containing at least one pharmaceutically acceptable excipient, comprising addition of an aqueous medium to at least one cyclodextrin and/or at least one of alpha-, beta- and gamma-cyclodextrin alkylated derivatives and subsequent addition of platinum complex of formula II to the thus-obtained solution of at least one cyclodextrin and/or at least one of alpha-, beta- and gamma-cyclodextrin alkylated derivatives in an aqueous medium; or addition of an aqueous medium to a mixture of platinum complex of formula II with at least one cyclodextrin and/or at least one of alpha-, beta- and gamma-cyclodextrin alkylated derivatives; or addition of at least one cyclodextrin and/or at least one of alpha-, beta- and gamma-cyclodextrin alkylated derivatives to a suspension of platinum complex of formula II in an aqueous medium.

Preferably, the aqueous medium is water for injections or an aqueous apyrogenic and sterile isotonic acetate, citrate or phosphate buffer, pH 4 to 8, preferably 4.

Within the framework of the invention, the term "aqueous medium" denotes water for injections or solutions of excipients in it, as accepted for intravenous application according to Pharmacopoeia. Preferred aqueous medium in a pharmaceutical composition according to the invention may be water or an aqueous buffer pH 4 to 8, such as a citrate or an acetate buffer.

The provided pharmaceutical compositions for administration by injection can be in the following forms:
a) dry injection form, comprising a mixture of at least one cyclodextrin and/or at least one of alfa-, beta- and gamma-cyclodextrin alkylated derivatives and optionally at least one pharmaceutically acceptable excipient, and which is ready for application upon addition of an aqueous medium;
b) liquid injection form which is ready for application and which is obtained either by addition of an aqueous medium to the dry injection form a), or by addition of an aqueous medium to at least one cyclodextrin and/or at least one of alfa-, beta- and gamma- cyclodextrin alkylated derivatives and subsequent addition of platinum complex of formula II to the obtained solution of at least one cyclodextrin and/or at least one of alfa-, beta- and gamma-cyclodextrin alkylated derivatives in an aqueous medium, or by addition of at least one cyclodextrin and/or at least one of alfa-, beta- and gamma- cyclodextrin alkylated derivatives to a suspension of platinum complex of formula II in an aqueous medium;
c) dry injection form which is obtained by lyophilization or spray-drying of the liquid injection form b), and which is ready for application upon addition of an aqueous medium;
d) liquid injection form which is ready for application and which is obtained by addition of an aqueous medium to the dry injection form c).

In case that pharmaceutically acceptable excipients are used in the pharmaceutical composition and such excipients are not already present in the dry injection form a), these excipients may be added at any stage of preparation of the above mentioned forms b) to d).

Such pharmaceutically acceptable excipients may be those that are commonly used in the preparation of injectable drug forms, e.g. isotonizing additives such as sodium chloride, or common antimicrobial additives such as e.g. benzoic acid derivatives (methyl- and propyl parabenes).

As cyclodextrins may be used preferably alfa-, beta- and gamma-cyclodextrins and their alkylated derivatives, such as particularly hydroxypropyl beta-cyclodextrin or hydroxypropyl methyl beta-cyclodextrin. In the inclusion complex, the tetravalent platinum complex is locked in the form of a partial or complete complex and such inclusion complex is practically indefinitely soluble in water. In this way, it is possible to prepare a solution of inclusion complex which is then sterilized by membrane filtration and is subsequently lyophilized or spray-dried, or simply to mix under aseptic conditions the platinum complex of formula II and the at least one cyclodextrin and/or derivatives the at least one of alfa-, beta- and gamma-cyclodextrin alkylated, and to prepare the inclusion complex "in situ" only during the preparation of the liquid injection form. The pharmaceutical composition for administration by injection is advantageously employed in the form of infusion. In each individual case, the resulting concentration and volume of such pharmaceutical composition intended for administration is determined by the attending physician.

In the following part the invention will be explained in more detail using specific examples of execution which are of illustrative value only and do not limit in any way the scope of the invention that is unequivocally defined by the appending Claims.

In these examples the platinum complex of formula II is denoted by code name LA-12

### Examples

### Example 1

### Preparation of dry injection form of platinum complex LA-12

Platinum complex LA-12 (1 part by weight) is mixed aseptically with beta-cyclodextrin of high purity (HP-beta-cyclodextrin; 3 parts by weight). The obtained mixture is then aseptically filled into sterile depyrogenized vials in a laminar flow box of A class purity.

### Example 2

### Preparation of lyophilized injection form of platinum complex LA-12

HP-beta-Cyclodextrin (3 parts by weight) is dissolved in 40 parts by weight of water for injections whereupon platinum complex LA-12 (1 part by weight) is gradually added to this solution under constant stirring. Stirring is continued until the formed inclusion complex completely dissolves. Then the solution is sterilized by membrane filtration (membrane pore size 0.22 µm), filled into vials and subjected to lyophilization.

### Example 3

### Preparation of dry injection form of platinum complex LA-12

HP-beta-Cyclodextrin (3 parts by weight) is dissolved in water for injections (40 parts by weight) whereupon platinum complex LA-12 (1 part by weight) is gradually added to this solution under constant stirring. Stirring is continued until the formed inclusion complex completely dissolves. Then the solution is sterilized by membrane filtration (membrane pore size 0.22 µm) and then it is aseptically spray-dried. The powder obtained is aseptically filled into sterile depyrogenized vials in a laminar flow box of A class purity.

### Example 4

### Preparation of lyophilized injection form of platinum complex LA-12

Platinum complex LA-12 (1 part by weight) is suspended in water for injections (40 parts by weight) whereupon HP-beta-cyclodextrin (3 parts by weight) is gradually added to this suspension under constant stirring, and stirring is continued until the formed inclusion complex completely dissolves. Then, the obtained solution is sterilized by membrane filtration (membrane pore size 0.22 µm) whereupon the sterile solution is filled into vials and is subjected to lyophilization.

### Example 5

### Preparation of dry injection form of platinum complex LA-12

Platinum complex LA-12 (1 part by weight) is suspended in water for injections (40 parts by weight). HP-beta-Cyclodextrin (3 parts by weight) is then added to the obtained suspension under constant stirring, and stirring is continued until the formed inclusion complex completely dissolves. The obtained solution is then sterilized by membrane filtration (membrane pore size 0.22 µm) whereupon it is aseptically spray dried. The obtained powder is then aseptically filled into sterile depyrogenized vials in a laminar flow box of A class purity.

## Claims

1. A method for manufacturing a pharmaceutical composition for administration by injection consisting of an aqueous solution of an inclusion complex of a platinum complex of formula II 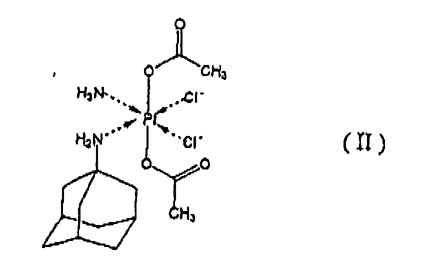 with at least one cyclodextrin and/or at least one of alpha-, beta- and gamma-cyclodextrin alkylated derivatives and optionally containing at least one pharmaceutically acceptable excipient, **characterized in that** it comprises addition of an aqueous medium to at least one cyclodextrin and/or at least one of alpha-, beta- and gamma-cyclodextrin alkylated derivatives and subsequent addition of platinum complex of formula II to the thus-obtained solution of at least one cyclodextrin and/or at least one of alpha-, beta- and gamma-cyclodextrin alkylated derivatives in an aqueous medium; or addition of an aqueous medium to a mixture of platinum complex of formula II with at least one cyclodextrin and/or at least one of alpha-, beta- and gamma- cyclodextrin alkylated derivatives; or addition of at least one cyclodextrin and/or at least one of alpha-, beta- and gamma-cyclodextrin alkylated derivatives to a suspension of platinum complex of formula II in an aqueous medium.

2. The method according to claim 1, **characterized in that** the aqueous medium is water or an aqueous buffer, pH 4 to 8, preferably 4.

## Patentansprüche

1. Ein Verfahren zur Herstellung einer pharmazeutischen Zusammensetzung zur Verabreichung durch Injektion, bestehender aus einer wässrigen Lösung eines Einschlusskomplexes aus einem Platin-Komplex der Formel II 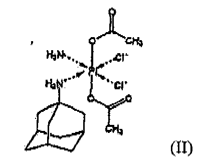 mit mindestens einem Cyclodextrin und/oder mindestens einem von den mit alpha-, beta- und gamma-Cyclodextrin alkylierten Derivaten und gegebenenfalls enthaltender mindestens einen pharmazeutisch annehmbaren Exzipienten, **dadurch gekennzeichnet, dass** es
Zugabe eines wässrigen Mediums zu mindestens einem Cyclodextrin und/oder mindestens einem von den mit alpha-, beta- und gamma-Cyclodextrin alkylierten Derivaten, und anschließende Zugabe von Platin-Komplex der Formel II zu der so erhaltenen Lösung von mindestens einem Cyclodextrin und/oder wenigstens von einem von den mit alpha-, beta-, und gamma-Cyclodextrin alkylierten Derivaten in ein wässriges Medium,
oder Zugabe eines wässrigen Mediums zu einer Mischung des Platin-Komplexes der Formel II mit mindestens einem Cyclodextrin, und/oder mindestens einem von den mit alpha-, beta-, und gamma-Cyclodextrin alkylierten Derivaten,
oder Zugabe von mindestens einem Cyclodextrin, und / oder mindestens einem von den mit alpha-, beta-, und gamma-Cyclodextrin alkylierten Derivaten zu einer Suspension von Platin-Komplex der Formel II in einem wässrigen Medium
umfasst.

2. Das Verfahren nach Anspruch 1, **dadurch gekennzeichnet, dass** das wässrige Medium Wasser oder ein wässriger Puffer mit pH 4 bis 8, vorzugsweise 4, ist.

## Revendications

1. Un procédé pour la production d'une composition pharmaceutique pour l'administration par injection se composant d'une solution aqueuse d'un complexe d'inclusion d'un complexe de platine de la formule II 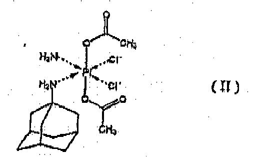 avec au moins un cyclodextrine et/ou au moins un derivé des cyclodextrines alpha-, beta- et gamma-alkylés et éventuellement contenant au moins un vehikulum pharmaceutiquement acceptable, **caractérisé en ce qu'**il renferme soit l'adjonction d'un milieu aqueux à un au moins cyclodextrine et/ou au moins un derivé des cyclodextrines alpha-, beta- et gamma-alkylés et l'addition successive du complexe de platine de la formule II à la solution tellement obtenue d'au moins un cyclodextrine et/ou d'au moins un derivé des cyclodextrines alpha-, beta- et gamma-alkylés dans le milieu aqueux; soit l'adjonction du milieu aqueux à un mélange du complexe de platine de la formule II avec au moins un cyclodextrine et/ou au moins un derivé des cyclodextrines alpha-, beta- et gamma-alkylés; soit l'adjonction d'au moins un cyclodextrine et/ou d'au moins un derivé des cyclodextrines alpha-, beta- et gamma-alkylés à une suspension du complexe de platine de la formule II dans le milieu aqueux.

2. Le procédé selon la revendication 1, **caractérisé en ce que** le milieu aqueux est l'eau ou un tampon aqueux, pH 4 à 8, de manière préférée 4.
